(19) **Europäisches Patentamt European Patent Office Office européen des brevets**

(11) **EP 4 495 581 B1**

(12) **FASCICULE DE BREVET EUROPEEN**

(45) Date de publication et mention
de la délivrance du brevet:
**31.12.2025 Bulletin 2026/01**

(51) Classification Internationale des Brevets (IPC):
*G01N 21/27* (2006.01) *G01N 21/33* (2006.01)
*G01N 33/00* (2006.01) *G01N 33/24* (2006.01)

(21) Numéro de dépôt: **24186169.9**

(22) Date de dépôt: **03.07.2024**

(52) Classification Coopérative des Brevets (CPC):
**G01N 21/272; G01N 21/33; G01N 33/24;**
G01N 2201/1211

(54) **PROCEDE POUR LE SUIVI DANS LE TEMPS DE LA CONCENTRATION EN UN COMPOSE CHIMIQUE D'UN FLUIDE, AU MOYEN D'UN SYSTEME DE MESURE OPTIQUE ET D'UN CAPTEUR DE TEMPERATURE**

VERFAHREN ZUR ZEITLICHEN ÜBERWACHUNG DER KONZENTRATION EINER CHEMISCHEN VERBINDUNG EINES FLUIDS MITTELS EINES OPTISCHEN MESSSYSTEMS UND EINES TEMPERATURSENSORS

METHOD FOR MONITORING THE CHEMICAL COMPOUND CONCENTRATION OF A FLUID IN TIME, BY MEANS OF AN OPTICAL MEASURING SYSTEM AND A TEMPERATURE SENSOR

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **18.07.2023 FR 2307665**

(43) Date de publication de la demande:
**22.01.2025 Bulletin 2025/04**

(73) Titulaire: **IFP Energies nouvelles**
**92500 Rueil-Malmaison (FR)**

(72) Inventeurs:
• **DESCHAMPS, Herve**
**92852 RUEIL-MALMAISON CEDEX (FR)**

• **BOUJLEL YOUNSI, Jalila**
**92852 Cedex RUEIL-MALMAISON (FR)**
• **YOUSSEF, Souhail**
**92852 RUEIL-MALMAISON CEDEX (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**EP-A1- 3 139 151 US-A1- 2022 003 662**

## Description

## Domaine technique

La présente invention concerne le domaine du suivi de l'évolution dans le temps d'une concentration en un composé chimique d'un fluide en circulation dans un milieu, au moyen d'une mesure d'absorbance en fonction de la longueur d'onde et d'une mesure de la température du fluide. Notamment, la présente invention trouve une application particulière pour le suivi de l'évolution dans le temps de la concentration en un composé chimique d'un fluide dans le cas où la température du fluide est variable dans le temps.

Par exemple, dans le domaine des géosciences (géothermie, stockage de $CO_2$, récupération assistée de pétrole, ...), des mesures en laboratoire sont souvent réalisées pour doser un composé chimique présent dans un fluide en circulation dans un échantillon de roche, afin d'identifier les éventuelles interactions / échanges qui ont eu lieu entre le fluide et le milieu poreux. De telles études visent à étudier et à comprendre les phénomènes physiques et géochimiques impliqués dans la roche.

Jusqu'à récemment, les dosages (de sels, tensioactifs, minéraux, huiles, polymères,...) sont réalisés en collectant les effluents dans des tubes (à l'aide des collecteurs de fractions) qui sont ensuite analysés manuellement et de manière différée dans le temps. Cette méthode présente plusieurs inconvénients :

- Analyse chronophage en termes de temps (mesure manuelle) ;
- Risque de vieillissement / altération des solutions avant dosage ;
- Mesures moyennées sur des gros volumes d'échantillons souvent exigés par la technique de mesure ;
- Résultat de mesure différé, ne pouvant pas permettre l'adaptation en temps réel de l'expérience de mesure si besoin ;
- Faible fréquence de mesure.

Le suivi d'une concentration en un composé chimique est de plus en plus réalisé au moyen de spectromètres équipés de cellules adaptées à la mesure en ligne (c'est-à-dire en continu, ou encore en temps réel) et pouvant couvrir différents types d'analyse (dosage, suivi de cinétique de réaction, turbidité, ...) de solutions d'intérêt pour les thématiques des géosciences (par exemple, interaction des sels, minéraux, tensioactifs, et/ou polymères avec la roche).

En particulier, la spectroscopie ultraviolet-visible (appelée souvent spectroscopie UV-VIS) fait partie des méthodes les plus utilisées pour la caractérisation des fluides et le dosage des espèces chimiques présentes. La spectrométrie UV-Vis est une technique de spectroscopie mettant en jeu les photons dont les longueurs d'ondes sont dans le domaine des ultraviolets (200 nm - 400 nm) et du visible (400 nm - 800nm). Soumises à un rayonnement dans cette gamme de longueurs d'onde, les molécules absorbantes subissent une transition électronique. La mesure de l'absorbance (ou densité optique) due à cette transition pour chaque longueur d'onde permet d'obtenir le spectre UV-Vis de la solution qui se définit comme la variation de l'absorbance en fonction des différentes longueurs d'onde.

L'analyse de ce spectre d'absorption permet d'accéder à des informations qualitatives, en détectant la présence de certaines substances mais surtout quantitatives en déterminant la concentration des espèces absorbantes.

## Technique antérieure

Le document suivant sera cité au cours de la description :
Malik, M., Chan, K. H., & Azimi, G. (2021). Quantification of nickel, cobalt, and manganese concentration using ultraviolet-visible spectroscopy. RSC Advances, 11(45), 28014-28028.

Parmi les méthodes d'analyse quantitatives, on connait des méthodes basées sur la loi de Beer-Lambert. La loi de Beer-Lambert est une relation empirique qui établit que, à une longueur d'onde $\lambda$ donnée, l'absorbance $A_\lambda$ d'une solution est proportionnelle à la concentration c des espèces absorbantes, ainsi qu'à la longueur du trajet ou chemin optique d (distance sur la laquelle la lumière traverse l'échantillon). Plus précisément, pour une solution limpide contenant une seule espèce absorbante cette loi s'écrit :

$$A_\lambda = \varepsilon_\lambda . d . c \ (1)$$

où $\varepsilon_\lambda$ est le coefficient d'extinction pour la longueur d'onde considérée. Ce coefficient ne dépend pas de la concentration de la solution ni de l'épaisseur traversée par la lumière ; en revanche, il dépend de la nature de la solution (espèce absorbante et solvant), et de la température.

En conditions ambiantes (température autour de 20°C), l'effet de la température est souvent négligé. Mais de manière générale, à une température T donnée, le spectre d'une solution contenant une seule espèce qui absorbe dans la gamme de longueur d'onde considérée présente une absorbance maximale Amax (T) à une longueur d'onde maximale λmax (T). Le couple (λmax ; Amax) caractérise l'espèce chimique absorbante à la température T. Classiquement, pour ce type de solutions mono-composants (c'est-à-dire ne comprenant qu'un seul composé chimique qui répond dans l'UV-Vis), pour déterminer la concentration d'un composé donné, la longueur d'onde choisie est la longueur d'onde λmax du pic Amax du spectre d'absorption (cf. par exemple le document Malik et al., 2021). Ce choix permet certes de minimiser l'incertitude sur l'absorbance, mais en raison de la loi logarithmique reliant l'absorbance à l'intensité lumineuse, des lumières parasites et d'autres phénomènes de diffusion et de fluorescence, il limite considérablement la dynamique de la mesure (gamme de concentrations mesurable).

Par ailleurs, un phénomène de saturation est observé au-delà d'une concentration critique dont la valeur dépend du composé à analyser. Plus précisément, au-delà d'une concentration critique, l'absorbance n'évolue plus d'une manière linéaire par rapport à la concentration et tend vers une même valeur maximale quelle que soit la concentration en place, rendant impossible de différencier les concentrations des solutions dont les concentrations excèdent cette valeur critique. Autrement dit, au-delà d'une concentration critique, l'absorbance sature et devient indépendante de la concentration.

Ainsi, l'utilisation de cette méthode d'analyse basée sur la loi de Beer-Lambert est classiquement restreinte aux solutions de faibles concentrations, ce qui est très limitant. Pour les fortes concentrations, on peut réaliser au préalable une dilution, mais cette étape, bien qu'elle reste envisageable pour des mesures ponctuelles, alourdit de manière considérable la procédure de mesure lorsqu'il s'agit d'un dosage en ligne où des milliers de mesures sont à réaliser sur des concentrations souvent inconnues. A cela s'ajoute le fait que la dilution est une source d'erreur supplémentaire sur la mesure.

Ainsi, cette méthode d'analyse basée sur la loi de Beer-Lambert est limitée aux solutions monocomposants très diluées, lorsqu'elles sont utilisées en ligne, pour deux raisons :

- dynamique de mesure (gamme de concentration) réduite en raison de la loi logarithmique.
- besoin d'automatisation du traitement des données pour l'analyse en ligne. En effet des centaines voire des milliers de spectres sont souvent à analyser.

On connait notamment la demande de brevet FR 22/01215 (numéro de dépôt) qui concerne un procédé pour déterminer une évolution dans le temps d'une concentration en un composé chimique d'un fluide, au moyen d'un système de mesure optique. Plus précisément, dans ce procédé, on construit un modèle de l'évolution de l'absorbance en fonction de la concentration de la manière suivante : on mesure une pluralité de spectres d'absorption du composé chimique correspondant chacun à une concentration du composé chimique, on définit une courbe intersectant chacun des spectres d'absorption en un seul point et telle que la courbe soit une fonction bijective de l'absorbance et de la longueur d'onde, on construit le modèle de l'évolution de l'absorbance en fonction de la concentration à partir des valeurs d'absorbance aux points d'intersection, et de la concentration relative à chaque spectre d'absorption. Puis, on détermine une évolution dans le temps d'une concentration du composé chimique au moyen du modèle ainsi déterminé. Toutefois, ce procédé ne permet pas un suivi dans le temps de la concentration du fluide lorsque la température du fluide est variable dans le temps. En effet, pour ce procédé, le modèle est construit à une température donnée, et ne peut être appliqué que dans le cas où la température du fluide reste constante dans le temps.

La présente invention permet de pallier ces inconvénients. Notamment, la présente invention concerne un procédé pour suivre dans le temps l'évolution d'une concentration en un composé chimique d'un fluide, au moyen d'un système de mesure optique et d'un capteur de température. Plus précisément, la présente invention concerne une méthode d'analyse de spectres d'absorption valide pour une gamme de concentrations et/ou une gamme de températures larges du fluide, tout en permettant une application en temps réel. En particulier, la présente invention ne nécessite pas d'avoir recours à une dilution, mais permet malgré tout un élargissement important de la gamme de mesure, sans compromettre la précision de la mesure. De plus, la présente invention permet un suivi de la concentration en un composé chimique d'un fluide dans le temps, même en cas de variations de la température du fluide dans le temps.

**Résumé de l'invention**

La présente invention concerne un procédé pour déterminer une évolution dans le temps d'une concentration en un composé chimique d'un fluide en circulation dans une zone de mesure, au moyen au moins d'un système de mesure optique pour mesurer une absorbance en fonction d'une longueur d'onde dudit fluide et d'un capteur de température pour mesurer une température dudit fluide, ledit composé chimique dudit fluide étant l'unique composé chimique dudit fluide ou l'unique composé chimique dudit fluide dont ladite concentration varie ou l'unique composé chimique dudit fluide ayant une absorbance non nulle dans une gamme de longueur d'ondes absorbées par ledit composé chimique, ledit procédé comprenant au moins les étapes suivantes :

A) pour chaque température prédéfinie d'une pluralité de températures prédéfinies, on construit un modèle intermédiaire de l'évolution de ladite absorbance en fonction de ladite concentration pour ladite température prédéfinie de la manière suivante :

i) au moyen au moins dudit système de mesure optique, on mesure une absorbance en fonction de la longueur d'onde pour une pluralité d'échantillons dudit fluide à ladite température prédéfinie et ayant des concentrations en ledit composé chimique distinctes, et on obtient une première pluralité de spectres d'absorption relatifs audit composé chimique correspondant chacun à une desdites concentrations en ledit composé chimique ;

ii) on définit une courbe intersectant chacun desdits spectres d'absorption de ladite première pluralité de spectres d'absorption en un seul point d'intersection et telle que ladite courbe soit une fonction bijective de ladite absorbance et de ladite longueur d'onde ;

iii) on construit ledit modèle intermédiaire de l'évolution de ladite absorbance en fonction de ladite concentration pour ladite température prédéfinie au moyen d'une régression linéaire appliquée à des premières valeurs d'absorbance auxdits points d'intersection entre ladite courbe et chacun desdits spectres d'absorption de ladite première pluralité de spectres d'absorption, et à ladite concentration correspondant à chacun desdits spectres d'absorption de ladite première pluralité de spectres d'absorption ;

B) à partir de coefficients directeurs et d'ordonnées à l'origine déterminés pour chacun desdits modèles intermédiaires de l'évolution de ladite absorbance en fonction de ladite concentration construits pour chacune desdites températures prédéfinies, on détermine par régression linéaire une première fonction représentative d'une variation dudit coefficient directeur en fonction de ladite température et une deuxième fonction représentative d'une variation de ladite ordonnée à l'origine en fonction de ladite température, et on construit ledit modèle de l'évolution de ladite absorbance en fonction de ladite concentration et de ladite température selon une formule du type :

$$A = Ma(T).\ln(C) + Mb(T)$$

où T est ladite température dudit fluide et C est ladite concentration dudit fluide ;

C) on détermine une évolution dans le temps d'une concentration en ledit composé chimique dudit fluide circulant dans ladite zone de mesure de la manière suivante :

a) au moyen au moins dudit système de mesure optique et dudit capteur de température, on mesure dans ladite zone de mesure respectivement une absorbance en fonction de la longueur d'onde et une température pour une succession de pas de temps et on obtient une deuxième pluralité de spectres d'absorption relatifs audit composé chimique correspondant chacun à un pas de temps ainsi qu'une température mesurée pour chaque pas de temps ; et

b) pour chacun desdits spectres d'absorption de ladite deuxième pluralité de spectres d'absorption, on détermine une deuxième valeur d'absorbance à l'intersection entre ladite courbe et ledit spectre d'absorption, et, au moyen dudit modèle de l'évolution de ladite absorbance en fonction de ladite concentration et de ladite température mesurée, et à partir de ladite deuxième valeur d'absorbance et de ladite température mesurée, on déduit ladite concentration en ledit composé chimique pour ledit pas de temps.

Selon une mise en œuvre de l'invention, ladite courbe peut être une droite.

Selon une mise en œuvre de l'invention, ledit système de mesure optique peut comprendre au moins une source lumineuse pour émettre un rayonnement dans au moins dans une gamme de longueur d'ondes prédéterminée, et un spectromètre pour mesurer une intensité lumineuse dudit rayonnement transmis au travers de ladite zone de mesure au moins dans ladite gamme de longueur d'ondes prédéfinie.

Selon une mise en œuvre de l'invention, ledit système de mesure optique peut comprendre en outre au moins une cellule de mesure reliée à ladite source lumineuse et audit spectromètre, dans laquelle peut se trouver ledit fluide.

Selon une mise en œuvre de l'invention, lorsque ledit composé chimique est l'unique composé chimique dudit fluide dont ladite concentration varie et en l'absence d'une étape de calibration dudit système de mesure optique au moyen d'un fluide de référence correspondant audit fluide à l'exclusion dudit composé chimique, on peut appliquer une étape de prétraitement aux mesures d'absorbance en fonction de la longueur d'onde pour déterminer un spectre d'absorption dudit composé chimique, comprenant au moins une soustraction dudit spectre d'absorption dudit composé chimique additionnel préalablement enregistré.

Selon une mise en œuvre de l'invention, ladite zone de mesure peut être disposée en aval d'un milieu poreux, tel qu'un échantillon d'une roche d'une formation souterraine, dans lequel circule ledit fluide.

Selon une mise en œuvre de l'invention, ledit composé chimique peut être choisi parmi la liste suivante : un tensio-actif, un sel, un composé hydrocarboné, un polymère.

Selon une mise en œuvre de l'invention, ledit procédé peut être mis en œuvre au moyen en outre d'un système de circulation de fluide pour faire circuler ledit fluide au moins dans ledit milieu poreux et ladite zone de mesure, ledit système de circulation de fluide comprenant une pompe, de préférence une pompe apte à délivrer un débit avec une haute précision, une cellule porte échantillon dans laquelle est disposé ledit milieu poreux, et de préférence un bain thermostaté ou une étuve pour contrôler la température dudit fluide dans ladite zone de mesure.

L'invention concerne en outre un système pour déterminer une évolution dans le temps d'une concentration en un composé chimique d'un fluide, ledit système comprenant une source lumineuse, un spectromètre, et des moyens pour le traitement et l'analyse de mesures réalisées par ledit spectromètre, ledit système étant apte à mettre en œuvre le procédé tel que décrit ci-dessus.

L'invention concerne en outre un produit programme d'ordinateur téléchargeable depuis un réseau de communication et/ou enregistré sur un support lisible par ordinateur et/ou exécutable par un processeur, comprenant des instructions de code de programme pour la mise en œuvre des étapes ii, et/ou iii), et/ou B), et/ou b), du procédé tel que décrit ci-dessus, lorsque ledit programme est exécuté sur un ordinateur.

D'autres caractéristiques et avantages du procédé et du système selon l'invention, apparaîtront à la lecture de la description ci-après d'exemples non limitatifs de réalisations, en se référant aux figures annexées et décrites ci-après.

**Liste des figures**

**[Fig 1]**

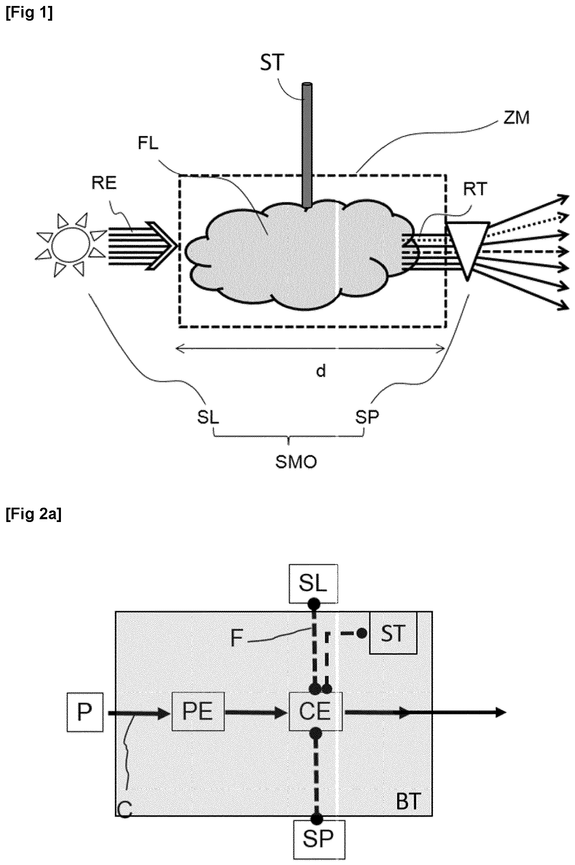

La figure 1 présente de manière schématique un mode de réalisation du système de mesure optique et d'un capteur de température aptes à la mise en œuvre du procédé selon l'invention.
**[Fig 2a]**
La figure 2a présente de manière schématique un mode de réalisation d'un système de mesure optique, d'un capteur de température et de moyens de circulation du fluide aptes à la mise en œuvre du procédé selon sa variante principale.
**[Fig 2b]**

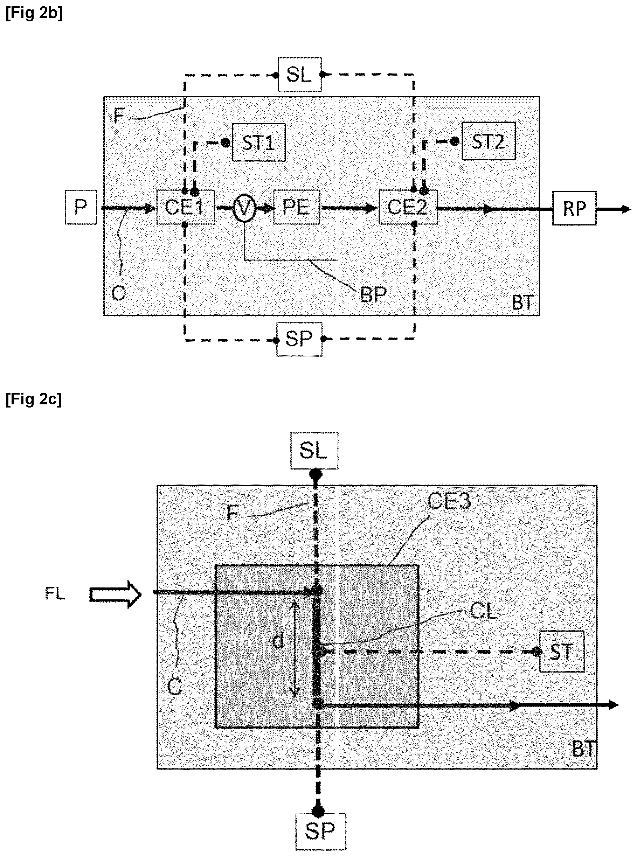

La figure 2b présente de manière schématique un autre mode de réalisation d'un système de mesure optique, d'un capteur de température et de moyens de circulation du fluide aptes à la mise en œuvre du procédé selon sa variante principale.
**[Fig 2c]**
La figure 2c présente un mode de réalisation d'une cellule de mesure pouvant être utilisée pour les modes de réalisation de la figure 2a ou de la figure 2b.
**[Fig 3]**

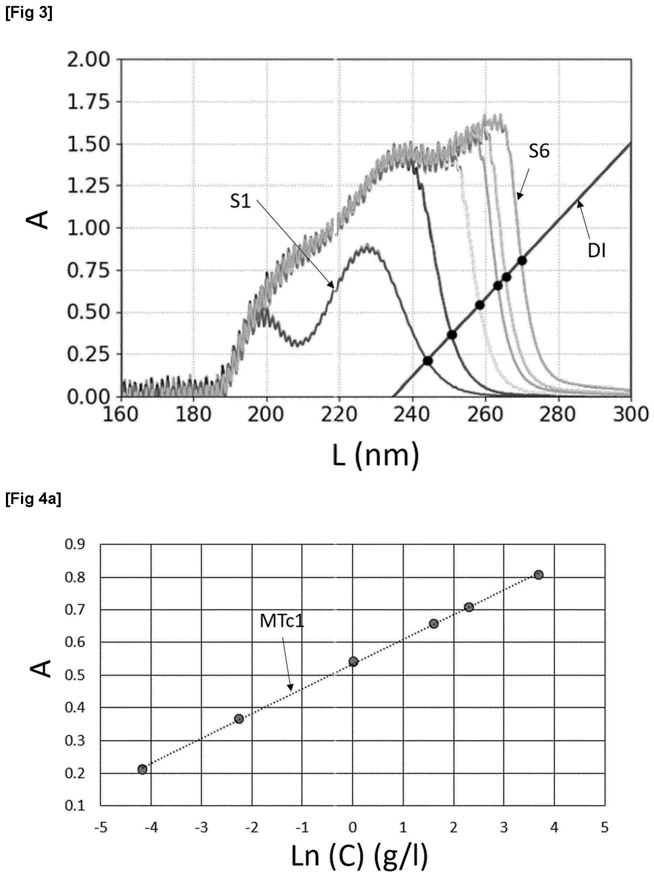

La figure 3 illustre, pour un premier exemple d'application, une pluralité de spectres d'absorption mesurés à une température prédéfinie lors de l'étape 1.1) du procédé selon l'invention décrite ci-après, ainsi que la courbe issue de l'étape 1.2) du procédé selon l'invention décrite ci-après.
**[Fig 4a]**
La figure 4a illustre, pour l'exemple d'application de la figure 3, un modèle intermédiaire de l'évolution de l'absorbance en fonction de la concentration construit lors de l'application de l'étape 1.3) du procédé selon l'invention décrite ci-après à la température prédéfinie de la figure 3.
**[Fig 4b]**

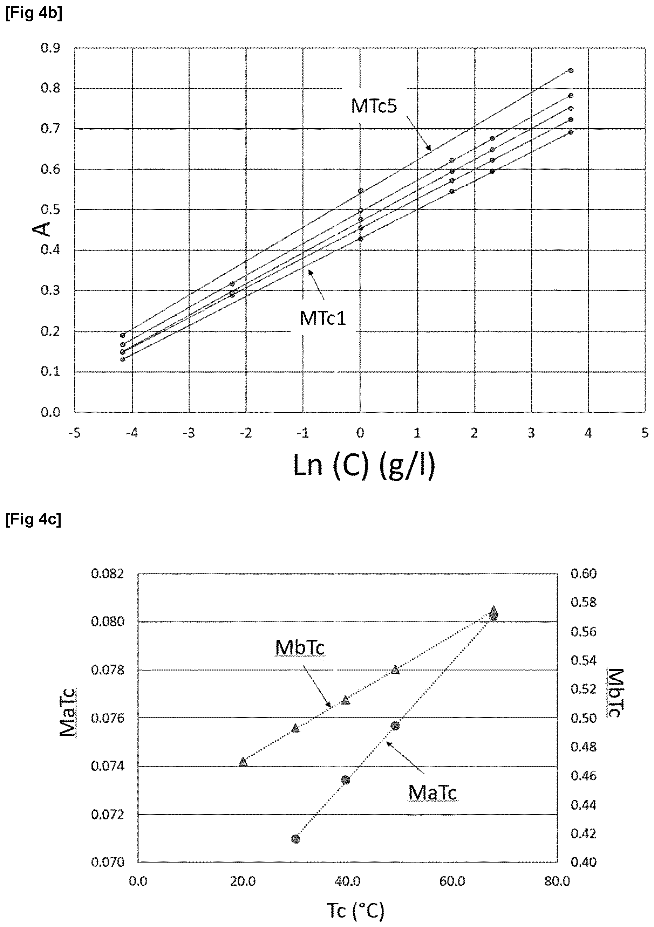

La figure 4b illustre, pour l'exemple d'application de la figure 3, cinq modèles intermédiaires de l'évolution de l'absorbance en fonction de la concentration construits par répétition des étapes 1.1) à 1.3) du procédé selon l'invention décrites ci-après, appliquées à cinq températures prédéfinies.
**[Fig 4c]**
La figure 4c illustre, pour l'exemple d'application de la figure 3, l'évolution des coefficients directeur et des ordonnées à l'origine en fonction de la température déterminés à partir des cinq modèles intermédiaires de la figure 4b.
**[Fig 5]**

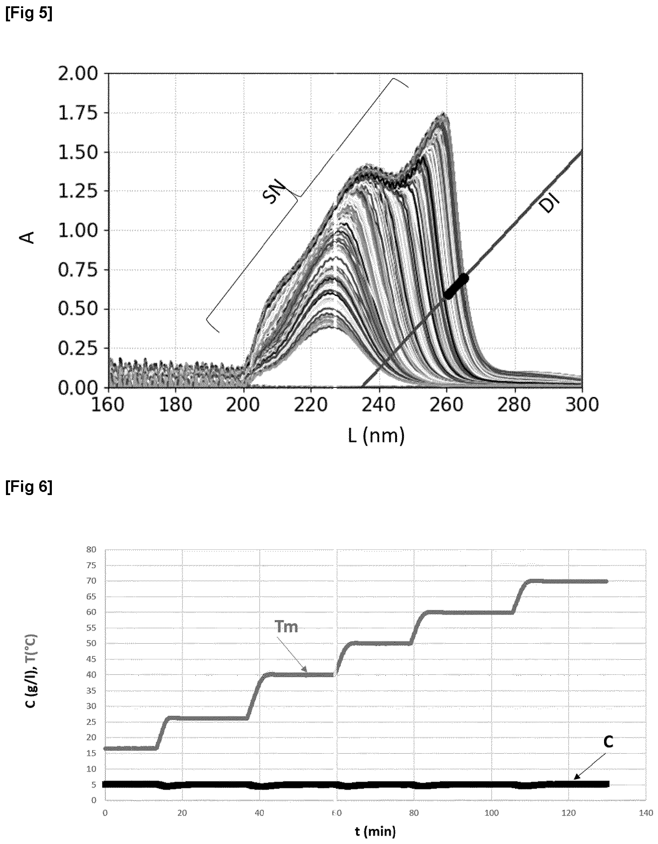

La figure 5 illustre, pour l'exemple d'application de la figure 3, une pluralité de spectres d'absorption mesurés lors de l'étape 3.1) du procédé selon l'invention décrite ci-après, ainsi que la courbe issue de l'étape 1.2) du procédé selon l'invention.
**[Fig 6]**
La figure 6 illustre, pour l'exemple d'application de la figure 3, une courbe représentant l'évolution dans le temps de la concentration en un composé chimique, issue de l'étape 3.2) du procédé selon l'invention décrite ci-après.
**[Fig 7]**

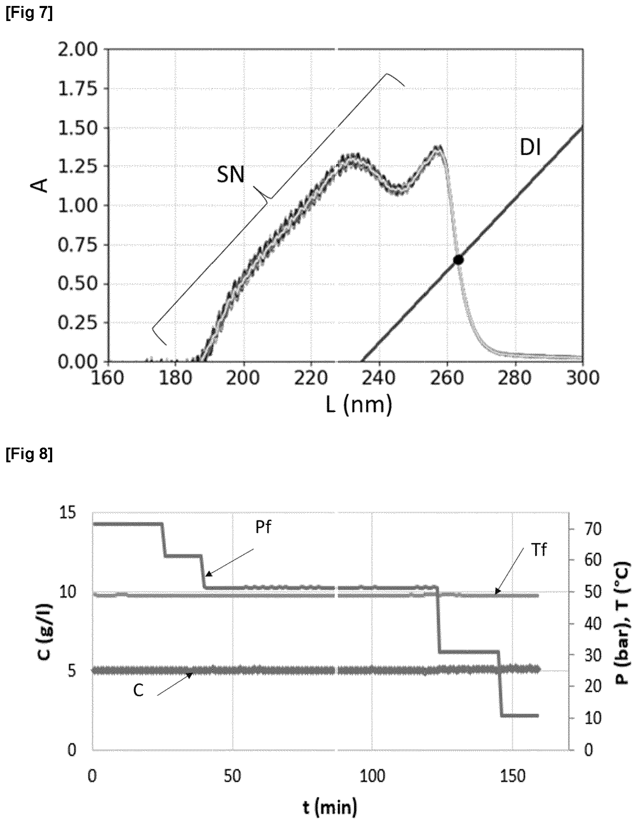

La figure 7 illustre une pluralité de spectres d'absorption mesurés lors de l'étape 1.1) du procédé selon l'invention décrite ci-après, appliquée à un deuxième exemple d'application.
**[Fig 8]**
La figure 8 illustre, pour l'exemple d'application de la figure 7, une courbe représentant l'évolution dans le temps de la concentration en un composé chimique, issue de l'étape 3.2) du procédé selon l'invention décrite ci-après.

**Description des modes de réalisation**

L'invention concerne un procédé pour mesurer une évolution dans le temps d'une concentration en un composé chimique d'un fluide circulant dans une zone de mesure, au moyen au moins d'un système de mesure optique pour mesurer une absorbance en fonction de la longueur d'onde et d'un capteur de température pour mesurer une température du fluide.
Selon une variante principale de l'invention pouvant trouver son application dans le domaine des géosciences, la zone de mesure peut être située en aval d'un échantillon d'un milieu poreux dans lequel on fait circuler le fluide comportant le composé chimique d'intérêt. En particulier, l'échantillon de milieu poreux peut être un échantillon d'une roche d'une formation souterraine, par exemple prélevé par carottage. Le procédé selon l'invention peut alors avoir pour objectif de suivre l'évolution de la concentration en le composé chimique d'intérêt en sortie de l'échantillon de milieu poreux dans lequel on injecte une solution comprenant le composé chimique selon une concentration connue, afin de comprendre les interactions fluide/roche. Selon une mise en œuvre, le procédé peut être utilisé pour suivre l'évolution dans le temps d'une réaction chimique, en suivant l'évolution de la concentration d'un composé chimique d'une solution suite à l'ajout d'un réactif en amont de la zone de mesure. Une application particulière peut être la détermination du pH d'une solution qui ne répond pas dans l'UV, en utilisant un réactif spécifique répondant dans l'UV.

Selon une alternative, la zone de mesure peut être aussi une portion de l'air ambiant d'un espace confiné ou semi-confiné de manière à suivre l'évolution d'une concentration en polluant. Il est bien clair que ces espaces confinés ou semi-confinés doivent être contrôlés en température.

Selon l'invention, le composé chimique dont on cherche à suivre l'évolution de la concentration dans le temps dans le fluide d'intérêt est soit :

- l'unique composé chimique du fluide d'intérêt : autrement dit, le fluide considéré ne comprend qu'un unique composé chimique (on parle alors de fluide mono-composant) ; ou
- l'unique composé chimique du fluide d'intérêt ayant une absorbance non (sensiblement) nulle dans la gamme de longueur d'ondes absorbées par le composé chimique considéré : autrement dit, le fluide considéré peut comprendre plusieurs composés chimiques (on parle alors de fluide multi-composants), mais tels que le spectre d'absorption du composé chimique d'intérêt soit dissocié des spectres d'absorption des autres composés chimiques (on parlera par la suite de composés chimiques additionnels). Par « sensiblement », on entend « au moins à l'erreur de mesure près ». Par « gamme de longueur d'ondes absorbées par le composé chimique », on entend la gamme de longueurs d'onde pour laquelle l'absorbance du composé chimique est non nulle. Autrement dit, pour cette alternative, les spectres d'absorption des composés chimiques additionnels présentent des valeurs d'absorbance (sensiblement) nulles pour les valeurs de longueurs d'onde pour lesquelles le spectre d'absorption du composé chimique d'intérêt présente des valeurs d'absorbance non (sensiblement) nulles ; ou
- l'unique composé chimique du fluide d'intérêt dont la concentration varie : autrement dit, là encore, le fluide considéré peut comprendre plusieurs composés chimiques, et les spectres d'absorption de ces composés chimiques additionnels peuvent se superposer au moins en partie au spectre d'absorption du composé chimique d'intérêt, mais leur concentration est invariante dans le temps.

Dans les deux premières alternatives décrites ci-dessus (fluide mono-composant et fluide multi-composants avec spectres d'absorption dissociés), aucun prétraitement du spectre d'absorption mesuré n'est nécessaire puisque le spectre d'absorption du composé chimique d'intérêt est directement accessible.

Dans la troisième alternative (fluide multi-composants avec spectres d'absorption pouvant se superposer, et tel que la concentration des composés additionnels est invariante dans le temps), on peut appliquer une étape de prétraitement au spectre d'absorption mesuré, telle qu'elle sera décrite ci-après, de manière à dissocier le spectre d'absorption du composé chimique d'intérêt du spectre d'absorption mesuré. En effet, le spectre d'absorption mesuré dans le cas d'un fluide multi-composants correspond à la somme des spectres d'absorption de l'ensemble des composés chimiques du fluide, selon la loi d'additivité des absorbances.

Selon une mise en œuvre de la variante principale de l'invention, le composé chimique d'intérêt peut être choisi parmi la liste suivante : un tensio-actif, un sel, un composé hydrocarboné, un polymère. Il s'agit en effet de composés chimiques d'intérêt en géosciences, notamment en géothermie, pour le stockage de $CO_2$, ou encore pour la récupération assistée de pétrole. Ces composés chimiques font souvent partie de fluides injectés dans une formation souterraine, que ce soit pour les stocker ($CO_2$, gaz naturel, etc.), ou bien en tant que fluide de balayage pour récupérer une ressource énergétique (hydrocarbures, chaleur, etc.) présente dans la formation souterraine poreuse. La connaissance de l'évolution dans le temps de la concentration en ces composés chimiques permet d'identifier les éventuelles interactions / échanges qui ont eu lieu entre le fluide injecté et le milieu poreux.

Selon une mise en œuvre de l'invention, le système de mesure optique peut comprendre :

- une source lumineuse pour émettre un rayonnement au moins dans une gamme de longueurs d'onde d'intérêt pour le composé chimique d'intérêt. Par gamme de longueurs d'onde d'intérêt, on entend une gamme de longueurs d'onde dans laquelle le spectre d'absorption caractéristique du composé chimique d'intérêt a des valeurs d'absorbance non nulles. Selon une mise en œuvre de la variante principale de l'invention selon laquelle le composé chimique d'intérêt est un tensio-actif, un sel, un composé hydrocarboné, ou un polymère, la gamme de longueurs d'onde de la source lumineuse peut être la gamme de longueurs d'onde des ultra-violets (notés UV), et
- un spectromètre pour mesurer une intensité lumineuse en fonction de la longueur d'onde dans au moins la gamme de longueurs d'onde d'intérêt pour le composé chimique d'intérêt.

La figure 1 présente de manière schématique un mode de réalisation du système de mesure optique SMO selon l'invention, comprenant une source lumineuse SL pour émettre un rayonnement RE, dans une zone de mesure ZM comprenant un fluide FL, et un spectromètre SP pour mesurer l'absorbance en fonction de la longueur d'onde du rayonnement RT ayant traversé le fluide FL dans la zone de mesure ZM selon un chemin optique de longueur d. Cette figure présente en outre de manière schématique un capteur de température ST pour la mesure de la température du fluide FL dans la zone de mesure ZM.

Selon un exemple de mise en œuvre de l'invention, la source lumineuse peut être une source halogène-deutérium, tel que le modèle AvaLight-DH-S-BAL de la société Avantes (Pays-Bas) et/ou le spectromètre peut être un spectromètre fibré haute sensibilité, tel que le modèle AvaSpec-HS2048XL-EVO de la société Avantes (Pays-Bas), ou toute source lumineuse analogue.

Avantageusement, le système de mesure optique peut comprendre une cellule de mesure reliée en amont à la source lumineuse et en aval (les directions amont et aval étant considérées par rapport au rayonnement émis par la source lumineuse) au spectromètre, et dans laquelle se trouve le fluide dont on souhaite mesurer une absorbance en fonction de la longueur d'onde. Selon une mise en œuvre, la cellule de mesure peut comporter deux entrées et deux sorties, pour permettre la liaison avec la source lumineuse et le spectromètre, mais aussi avec un système de circulation de fluide décrits ci-après.

Avantageusement, le système de mesure optique peut comprendre des moyens pour le traitement et l'analyse de l'intensité lumineuse mesurée par le spectromètre, afin de déterminer un spectre d'absorption à partir de l'intensité lumineuse mesurée. Selon un exemple de mise en œuvre, les moyens pour le traitement et l'analyse de l'intensité lumineuse mesurée par le spectromètre peuvent comprendre un ordinateur sur lequel est installé le logiciel Avasoft de la société Avantes (Pays-Bas) pour déterminer les spectres d'absorption à partir de l'intensité lumineuse mesurée. Avantageusement, le système de mesure optique peut comprendre des fibres optiques de 400 µm de cœur pour relier la source lumineuse à la cellule de mesure, et la cellule de mesure au spectromètre. Avantageusement, le système de mesure optique peut comprendre en outre des moyens pour la transmission (par exemple par voie filaire électrique, par fibre optique ou par un système de communication sans fil) des mesures réalisées par le spectromètre aux moyens pour le traitement et l'analyse de l'intensité lumineuse.

Selon une mise en œuvre de l'invention, le procédé peut comprendre une étape, éventuellement préalable, de calibration du système de mesure optique. Une telle étape de calibration peut comprendre une mesure d'une intensité lumineuse en fonction de la longueur d'onde transmise au travers d'un fluide de référence. Selon une mise en œuvre selon laquelle le fluide d'intérêt est un liquide mono-composant, on peut utiliser de l'eau purifiée en tant que fluide de référence. Selon les alternatives selon laquelle le fluide d'intérêt comporte, en plus du composé chimique d'intérêt, des composés chimiques additionnels tels que définis ci-dessus, le fluide de référence peut ne contenir que les éléments chimiques additionnels, présents selon leurs concentrations respectives dans le fluide d'intérêt. Selon une réalisation de cette mise en œuvre de l'invention, l'étape de calibration peut comprendre les étapes suivantes :

- l'émission par la source lumineuse d'un rayonnement à travers le fluide de référence au sein d'une zone de mesure ;
- la détection par le spectromètre du rayonnement ayant traversé le fluide de référence dans la zone de mesure et la génération d'une intensité lumineuse en fonction de la longueur d'onde du rayonnement ayant traversé le fluide de référence.

A partir de cette calibration, l'absorbance A d'un fluide peut être déterminée selon une formule du type :

$$A(\lambda) = -\ln\left(\frac{I_s(\lambda)}{I_0(\lambda)}\right)(2),$$

où $\lambda$ est la longueur d'onde, $I_s(\lambda)$ est l'intensité lumineuse en fonction de la longueur d'onde du rayonnement transmis au travers du fluide considéré, et $I_0(\lambda)$ est l'intensité lumineuse en fonction de la longueur d'onde du rayonnement transmis au travers du fluide de référence.

Il est bien clair que pour l'application de l'étape 1) décrite ci-après, l'étape préalable de calibration du système de mesure optique est réalisée pour la pluralité de températures prédéfinies de l'étape 1).

Selon une mise en œuvre au moins de la variante principale de l'invention, le procédé peut être mis en œuvre en outre au moyen d'un système de circulation de fluide comprenant :

- une pompe, de préférence une pompe apte à délivrer un débit avec une haute précision (comme par exemple une pompe pour chromatographie liquide de haute performance, dites pompes HPLC), de préférence pouvant délivrer un débit dans la gamme 0 - 2 ml/min ;
- une cellule porte échantillon, dans laquelle est placé le milieu poreux,
et, optionnellement,
- un régulateur de pression permettant d'imposer la pression de ligne dans tout le circuit fluidique ; et/ou
- un bain thermostaté ou une étuve, pour le contrôle de la température du fluide dans la zone de mesure.

Avantageusement, le système de circulation de fluide dans le milieu poreux peut comprendre en outre :

- un débitmètre pour contrôler le débit de la pompe ; et/ou

- un capteur de pression différentielle permettant de mesurer la perte de charge le long du milieu poreux ; et/ou

- des moyens de connexion et de contrôle du fluide, tels que des vannes, des conduites ; et/ou

- un capteur de température supplémentaire (en plus du capteur de température selon l'invention) ; et/ou

- un by-pass, par exemple sous la forme d'une conduite, permettant de contourner le milieu poreux ; et/ou

- des moyens pour le traitement et l'analyse des mesures de débit, et/ou de pression et/ou de température. Avantageusement, les moyens pour le traitement et l'analyse des mesures de débit et/ou de pression et/ou de température peuvent être les mêmes que les moyens pour le traitement et l'analyse des mesures de l'intensité lumineuse réalisées au moyen d'un quelconque mode de réalisation du système optique.

Dans une mise en œuvre préférée du procédé selon l'invention, la cellule de mesure du système de mesure optique, le porte-échantillon, et optionnellement le by-pass du système de circulation du fluide, peuvent être plongés dans un bain thermostaté ou encore une étuve permettant de contrôler la température du fluide dans la zone de mesure. L'utilisation d'une étuve ou d'un bain thermostaté dans lequel la cellule de mesure et la roche sont stockées permet de s'assurer que le fluide dans la cellule de mesure présente la même température que celui en circulation dans la roche. Des alternatives peuvent être mises en œuvre, comme par exemple contrôler la température du fluide injecté et réaliser l'injection dans la cellule de mesure à fort débit pour garantir que la température ne change pas entre le réservoir dans lequel le fluide est stocké et son arrivée dans la cellule de mesure.

La figure 2a présente un mode de réalisation d'un système de mesure optique et de moyens de circulation aptes à la mise en œuvre du procédé selon sa variante principale, comprenant une pompe P pour faire circuler, via des conduites C représentées par des flèches, le fluide d'intérêt dans un échantillon de roche (non représenté) disposé dans un porte échantillon PE, puis dans une cellule de mesure CE, qui est elle-même reliée à une source lumineuse SL et un spectromètre SP via des fibres optiques F, et à une sonde de température ST. De plus, dans cette conception, le porte-échantillon PE, la cellule de mesure CE et le by-pass BP sont plongés dans un bain thermostaté BT permettant le contrôle de la température du fluide. Une telle cellule de mesure CE permet une mesure optique et une mesure de température dans une zone de mesure dans laquelle circule un fluide.

La figure 2b présente un autre mode de réalisation d'un système de mesure optique et de moyens de circulation aptes à la mise en œuvre du procédé selon sa variante principale, se distinguant du mode de réalisation de la figure 2a en ce qu'il comprend deux cellules de mesures CE1, CE2 : une cellule de mesure CE1 disposée en amont de l'échantillon de roche (non représenté) disposé dans le porte-échantillon PE et une autre cellule de mesure CE2 disposée en aval de l'échantillon de roche. La cellule de mesure CE1 disposée en amont de l'échantillon a pour seul but de vérifier la stabilité de la solution injectée dans l'échantillon de roche, alors que la cellule de mesure CE2 disposée en sortie de l'échantillon de roche a pour but de doser les effluents en sortie du milieu. Les cellules de mesure CE1, CE2 sont chacune reliées à des sondes de température ST1, ST2, ainsi qu'à la source lumineuse SL et au spectromètre SP par des fibres optiques F. Le système de circulation de fluide comprend une pompe P en amont de la cellule de mesure amont CE1, des conduites C pour relier la pompe P à la cellule de mesure amont CE1, la cellule de mesure amont CE1 à l'échantillon disposé dans le porte-échantillon PE, et l'échantillon à la cellule de mesure aval CE2. Un régulateur de pression RP permettant d'imposer la pression de ligne dans tout le circuit fluidique est placé en sortie du montage. Le système de circulation de fluide comprend en outre un by-pass BP pour contourner l'échantillon de roche, contrôlé par une vanne V. Le by-pass BP est notamment utile pour réaliser l'application de l'étape 1.1) décrite ci-dessous pour chacune des deux cellules de mesure CE1, CE2, au moyen d'une unique injection d'un échantillon de fluide. De plus, dans cette conception, le porte-échantillon PE, les cellules de mesure CE1, CE2 et le by-pass BP sont plongés dans un bain thermostaté BT permettant le contrôle de la température du fluide. Ce mode de réalisation est particulièrement apte à une mise en œuvre du procédé selon l'invention dans le cas de conditions HPHT (Haute Pression Haute Température), comme cela sera démontré dans le deuxième exemple d'application ci-après. Cela peut être utile dans de nombreuses applications de géosciences (géothermie, stockage de $CO_2$, stockage d'hydrogène, ...) car la température du fluide en circulation dans une formation ainsi que la pression à laquelle il est soumis augmentent avec la profondeur et peuvent atteindre des valeurs très élevées. Afin d'étudier les interactions fluide / roche à l'échelle du laboratoire et décrire correctement les réactions chimiques impliquées, il est important de se placer dans des conditions de température et de pression représentatives de l'application concernée.

La figure 2c présente un mode de réalisation d'une cellule de mesure CE3, par exemple pouvant être utilisée pour les modes de réalisation de la figure 2a ou de la figure 2b, comprenant un canal CL de longueur d connecté aux conduites C dans lesquelles circulent le fluide FL, à un spectromètre SP et à une source lumineuse via des fibres optiques F, ainsi qu'à une sonde de température ST dont la section constituant l'extrémité affleure le fluide en circulation dans ce canal. Ce type de cellule de mesure CE3 permet de réaliser une mesure de l'absorbance en fonction de la longueur d'onde relative au fluide FL circulant dans le canal CL ainsi qu'une mesure de la température T de ce même fluide. Selon le mode de réalisation illustré, le canal CL est sensiblement perpendiculaires aux conduites C et à la sonde de température ST. De manière non limitative, sur cette figure, la température du fluide en circulation est contrôlée grâce à un bain thermostaté BT dans lequel la cellule de mesure CE3 est plongée. Avantageusement, on peut ajuster la longueur d du canal, en fonction de la gamme de concentrations à mesurer. En effet, cette longueur constitue la longueur du chemin optique dont dépend l'absorbance (cf. équation (1) ci-dessous) : plus le chemin optique est long, et plus on peut avoir accès à des faibles concentrations.

Le procédé selon l'invention comprend au moins les étapes suivantes décrites ci-après.

1) Construction d'une pluralité de modèles intermédiaires de l'évolution de l'absorbance en fonction de la concentration pour des températures prédéfinies.

Il s'agit ici de construire, pour chaque température prédéfinie d'une pluralité de températures prédéfinies, un modèle intermédiaire de l'évolution de l'absorbance en fonction de la concentration pour la température prédéfinie. Autrement dit, il s'agit de construire une pluralité de modèles intermédiaires de l'évolution de l'absorbance en fonction de la concentration, correspondant chacun à une température prédéfinie d'une pluralité de températures prédéfinies. Encore dit autrement, il s'agit ici de calibrer des modèles de l'évolution de l'absorbance en fonction de la concentration à différentes températures fixées.

Selon une mise en oeuvre de l'invention, le nombre de températures prédéfinies de la pluralité de températures prédéfinies vaut au moins 3, de préférence 5, très préférentiellement 10. Il est bien clair que la gamme des températures prédéfinies peut être choisie en fonction des températures minimales et maximales attendues lors de la mise en oeuvre de l'étape 3) du procédé selon l'invention. Par exemple, si le procédé selon l'invention est mis en œuvre dans le cadre d'une expérience de réinjection de fluide géothermique dans le Dogger (aquifère géothermique exploité en région parisienne), on prendra une gamme de températures prédéfinies avec des températures comprises entre 57 et 85°C (valeurs associées à celles mesurées sur site). Avantageusement, pour une gamme de températures prédéfinies donnée, on peut par exemple utiliser un pas de 5°C (notamment pour la gamme précitée, relativement étroite), ou bien un pas de 10°C entre chaque température prédéfinie. L'homme du métier sait choisir un pas ad hoc en fonction de la gamme de températures prédéfinie.

Les étapes 1.1) à 1.3) décrites ci-après sont mises en oeuvre pour chaque température prédéfinie de la pluralité de températures prédéfinies. Autrement dit, les étapes 1.1) à 1.3) sont répétées pour chaque température prédéfinie de la pluralité de températures prédéfinies. On parlera par la suite de température en cours pour une itération des étapes 1.1) à 1.3) à une température prédéfinie donnée.

### 1.1 Acquisition d'une pluralité de spectres d'absorption pour une pluralité de concentrations en le composé chimique d'intérêt et pour une température prédéfinie

Au cours de cette étape, au moyen au moins du système de mesure optique, on mesure une absorbance en fonction de la longueur d'onde pour une pluralité d'échantillons du fluide, les échantillons ayant des concentrations en le composé chimique d'intérêt distinctes et étant à la température prédéfinie, et on obtient une première pluralité de spectres d'absorption correspondant chacun à une des concentrations en le composé chimique d'intérêt. Autrement dit, cette étape vise à déterminer un spectre d'absorption (c'est-à-dire une courbe représentant l'évolution de l'absorbance en fonction de la longueur d'onde) pour chaque échantillon de fluide ayant une concentration en le composé chimique d'intérêt distincte d'un autre échantillon de la pluralité d'échantillons. Il est bien clair que la concentration en composé chimique de chaque échantillon est connue. La température est identique d'un échantillon à un autre, et égale à la température prédéfinie. Pour la mise en oeuvre de cette étape, on peut par exemple utiliser les moyens de circulation du fluide décrits dans les figures 2a ou 2b, qui permettent de contrôler la température du fluide au moyen d'un bain thermostaté. Avantageusement, le nombre d'échantillons de fluide ayant des concentrations en le composé chimique distinctes vaut au moins 5, et vaut de préférence 10, très préférentiellement 15. Il est bien clair que la gamme de concentrations couverte par les échantillons peut être choisie en fonction des concentrations attendues lors de la mise en oeuvre de l'étape 3). Par exemple dans le cas de la variante principale de l'invention, il est classique de vouloir suivre l'évolution dans le temps de la concentration en sortie d'un milieu poreux d'une solution de concentration en un composé chimique connue, injectée en entrée du milieu poreux. Ainsi, la gamme de concentrations couverte par les échantillons de fluide peut avantageusement varier entre une concentration nulle et la concentration de la solution qui sera en entrée du milieu poreux, de préférence entre une concentration nulle et une valeur maximale supérieure (par exemple de 50%) à la concentration de la solution en entrée du milieu poreux, pour être à même de mesurer des concentrations en sortie du milieu poreux excédant la concentration en entrée en raison d'un phénomène de rétention temporaire (par adsorption ou autre) dans le milieu poreux.

Selon les alternatives selon lesquelles le fluide considéré est un fluide mono-composant ou bien un fluide multi-composants avec des spectres dissociés tels que décrits ci-dessus, les courbes d'absorbance en fonction de la longueur d'onde mesurées correspondent directement aux spectres d'absorption du composé chimique d'intérêt.

Selon une mise en œuvre selon laquelle le fluide considéré comporte un ou des composés chimiques additionnels par rapport au composé chimique d'intérêt, dont les spectres d'absorption se superposent au moins en partie au spectre d'absorption du composé chimique d'intérêt et dont la concentration est connue et invariante dans le temps, on peut réaliser un prétraitement de la mesure de l'absorbance en fonction de la longueur d'onde afin d'obtenir le spectre d'absorption du composé chimique d'intérêt. Selon une réalisation de cette mise en œuvre de l'invention, on peut réaliser un prétraitement de la mesure de l'absorbance en fonction de la longueur d'onde de la manière suivante : pour chaque composé chimique additionnel, on mesure le spectre d'absorption d'un échantillon d'une solution comprenant ce composé chimique additionnel selon sa concentration connue dans le fluide d'intérêt, et on soustrait le spectre d'absorption de ce composé chimique additionnel au spectre d'absorption mesuré pour le fluide d'intérêt. A noter que, lorsqu'une étape de calibration préalable du système de mesure optique a été réalisée au moyen d'un fluide de référence formé des composés chimiques additionnels selon leurs concentrations respectives tel que décrit ci-dessus (c'est-à-dire pour un fluide de référence correspondant au fluide d'intérêt, à l'exclusion du composé chimique d'intérêt), il n'est pas nécessaire de réaliser un prétraitement de la mesure de l'absorbance en fonction de la longueur d'onde, puisque l'intensité lumineuse mesurée est de fait corrigée par l'intensité lumineuse du fluide de référence, selon la formule de l'équation (2).

Selon une mise en œuvre de la variante principale de l'invention, on peut mettre en œuvre l'étape 1.1) au moyen d'un système de mesure optique et d'un système de circulation de fluide tels que décrits dans la figure 2b, le système de circulation de fluide étant configuré tel que la vanne V permet de contourner le milieu poreux via le by-pass BP. De cette manière, on peut mesurer, avec un unique système de circulation de fluide, une absorbance en fonction de la longueur d'onde pour chaque échantillon de fluide et au niveau de chaque cellule de mesure CE1, CE2, afin de construire un modèle intermédiaire de l'évolution de l'absorbance en fonction de la concentration et à température fixée pour chaque cellule de mesure CE1, CE2 tel que décrit ci-dessous. Ce modèle est valable à la température prédéfinie en cours.

La figure 3 illustre une pluralité de spectres d'absorption issus de l'application de l'étape 1.1) à un exemple d'application qui sera décrit ci-après. En particulier, cette figure présente six courbes (seules les courbes S1 et S6 sont annotées pour des raisons de clarté) représentant l'évolution de l'absorbance A en fonction de la longueur d'onde L en nm, chaque courbe résultant d'une mesure réalisée pour un fluide ayant une concentration en un composé chimique unique donnée. De plus, tous les spectres ont été obtenus sur des échantillons présentant la même température prédéfinie (en l'espèce Tc1 = 50°C pour cette figure). Sur cette figure, le spectre d'absorption S1 correspond à la concentration la plus faible et le spectre S6 correspond à la concentration la plus élevée.

**1.2) Définition d'une courbe de l'absorbance en fonction de la longueur d'onde pour une température prédéfinie**

Au cours de cette étape, on définit une courbe intersectant en un seul point chacun des spectres d'absorption de la pluralité de spectres d'absorption mesurés à l'étape 1.1) et telle que cette courbe soit une fonction bijective de l'absorbance et de la longueur d'onde (autrement dit, cette courbe est telle qu'à toute valeur d'absorbance ne correspond qu'une seule valeur de longueur d'onde et réciproquement).

Autrement dit, on définit une courbe de l'absorbance en fonction de la longueur d'onde intersectant :

- l'ensemble des spectres d'absorption : cela permet de couvrir l'ensemble la gamme de concentrations prédéfinie ;
- en un seul point : cela permet de contribuer à l'unicité des valeurs de concentration déterminées lors de l'étape 3) décrite ci-dessous.
- et telle qu'à une valeur d'absorbance ne corresponde qu'une seule valeur de longueur d'onde et réciproquement : cela permet également de contribuer à l'unicité des valeurs de concentration déterminées lors de l'étape 3) décrite ci-dessous.

De manière très préférée, la courbe définie peut être une droite. Ce mode de mise en oeuvre est avantageux, car il est alors très rapide de déterminer l'intersection de la droite avec chacun des spectres d'absorption, notamment plus rapide qu'avec une courbe représentée par une fonction plus complexe, comme par exemple une fonction polynômiale de degré au moins 2. Cela permet de contribuer à une détermination en temps réel de l'évolution de la concentration en un composé chimique dans un milieu lors de l'étape 3) décrite ci-dessous. Mais il est bien clair que toute autre courbe répondant aux critères ci-dessus peut être définie.

La figure 3 décrite ci-dessus présente un exemple de courbe, sous la forme d'une droite DI, répondant aux critères ci-dessus appliqués aux spectres d'absorption S1, S6 mesurés à l'étape 1.1). On peut notamment observer que la droite DI ainsi définie intersecte en un seul point l'ensemble des spectres d'absorption S1, S6, et dans des portions de ces spectres où une seule valeur d'absorbance est associée à une seule valeur de longueur d'onde, et réciproquement.

Aux fins de l'étape 1.3) décrite ci-après, on peut avantageusement collecter les valeurs d'absorbance à l'intersection entre la courbe ainsi définie à l'étape 1.2) et chacun des spectres d'absorption de la pluralité de spectres d'absorption déterminés à l'issue de l'étape 1.1).

**1.3) Construction d'un modèle intermédiaire de l'évolution de l'absorbance en fonction de la concentration pour une température prédéfinie**

Au cours de cette étape, on construit un modèle intermédiaire de l'évolution de l'absorbance en fonction de la concentration pour une température prédéfinie à partir des valeurs d'absorbance aux points d'intersection entre la courbe définie à l'étape 1.2) et les spectres d'absorption déterminés à l'étape 1.1), et à partir de la concentration correspondant à chacun de ces spectres d'absorption.

En effet, chaque spectre d'absorption déterminé à l'étape 1.1) correspond à une concentration connue en le composé chimique d'intérêt. Pour un spectre d'absorption, on peut alors associer à la valeur d'absorbance au point d'intersection entre ce spectre et la courbe de l'étape 1.2), la concentration associée à ce spectre. On obtient ainsi pour chaque spectre d'absorption un couple formé par une absorbance et une concentration.

Selon l'invention, pour une température prédéfinie, on construit un modèle intermédiaire de l'évolution de l'absorbance en fonction de la concentration en recherchant par régression linéaire une fonction permettant d'approcher au mieux les valeurs d'absorbance aux points d'intersection entre la courbe définie à l'étape 1.2) et les spectres d'absorption déterminés à l'étape 1.1), en fonction de leurs valeurs de concentration associées. Le modèle intermédiaire de l'évolution de l'absorbance en fonction de la concentration pour une température prédéfinie est ainsi une droite.

La figure 4a présente un exemple de modèle MTc1 de l'évolution de l'absorbance A en fonction de la concentration C en g/l (en échelle logarithmique), déterminé par régression linéaire à partir des couples (représentés par des points sur cette figure) formés par une valeur d'absorbance et une valeur de concentration et obtenus à la température prédéfinie (notée Tc1 par la suite) du fluide.

A la fin de cette étape appliquée à une température prédéfinie donnée, on obtient de fait, par la régression linéaire, un coefficient directeur et une ordonnée à l'origine du modèle intermédiaire de l'évolution de ladite absorbance en fonction de la concentration construit pour la température prédéfinie en cours.

A l'issue de la répétition des étapes 1.1) à 1.3) pour chaque température prédéfinie de la pluralité de températures prédéfinies, on obtient un coefficient directeur et une ordonnée à l'origine pour chacun des modèles intermédiaire de l'évolution de ladite absorbance en fonction de la concentration construits pour la pluralité de températures prédéfinies.

La figure 4b présente un exemple de cinq modèles intermédiaires MTc1, MTc5 (seuls les modèles MTc1 et MTc5 sont annotés pour des raisons de clarté de la figure) d'évolution de l'absorbance A en fonction de la concentration C (en échelle logarithmique) relatifs à cinq valeurs de températures prédéfinies (notées Tc1 à Tc5 par la suite), construits par régression à partir des couples (représentés par des points sur cette figure) formés par une valeur d'absorbance A et une valeur de concentration C.

**2) Construction d'un modèle de l'évolution de l'absorbance en fonction de la concentration**

Au cours de cette étape, il s'agit de construire un modèle de l'évolution de l'absorbance en fonction de la concentration, à partir des coefficients directeurs et des ordonnées à l'origine déterminés pour chacun des modèles intermédiaires de l'évolution de l'absorbance en fonction de la concentration construits pour chacune des températures prédéfinies de l'étape 1).

Selon l'invention, on détermine par régression linéaire une première fonction Ma(T) représentative d'une variation du coefficient directeur en fonction de la température et une deuxième fonction Mb(T) représentative d'une variation de l'ordonnée à l'origine en fonction de la température, à partir des coefficients directeurs et des ordonnées à l'origine déterminés pour chacun des modèles intermédiaires de l'évolution de l'absorbance en fonction de la concentration construits pour chacune des températures prédéfinies, c'est-à-dire à partir des coefficients directeurs et des ordonnées à l'origine déterminés pour chacune des températures prédéfinies.

Selon l'invention, on construit alors le modèle de l'évolution de l'absorbance en fonction de la concentration et de la température selon une formule du type :

$$A = Ma(T).\ln(C) + Mb(T) \qquad (3)$$

où T est la température du fluide et C est la concentration du fluide. Autrement dit, on détermine un modèle de l'évolution de l'absorbance en fonction de la concentration dont les coefficients varient en fonction de la température du fluide. A partir d'une telle formule, quelle que soit la température du fluide, on peut déterminer la concentration du fluide à partir d'une mesure d'absorbance.

La figure 4c illustre l'évolution du coefficient directeur MaTc et de l'ordonnée à l'origine MbTc en fonction de la température prédéfinie Tc des modèles intermédiaires de la figure 4b déterminés pour les températures prédéfinies Tc1 à Tc5.

**3) Détermination de l'évolution dans le temps de la concentration en le composé chimique**

Le modèle de l'évolution de l'absorbance en fonction de la concentration et de la température ayant été déterminé à l'issue de l'étape précédente, ce modèle peut être utilisé pour déterminer une évolution dans le temps de la concentration en le composé chimique d'intérêt du fluide circulant dans une zone de mesure à une température donnée, qui peut être variable ou constante.

### 3.1) Acquisition de spectres d'absorption pour une succession de pas de temps

Au moins au moyen du système de mesure optique et du capteur de température tels que décrits ci-dessus, on mesure dans la zone de mesure (dans laquelle circule le fluide comprenant lui-même le composé chimique d'intérêt) la température du fluide, ainsi qu'une absorbance en fonction de la longueur d'onde pour une succession de pas de temps, et on obtient une deuxième pluralité de spectres d'absorption relatifs audit composé chimique correspondant chacun à un pas de temps et à une température.

Autrement dit au cours de cette étape, on mesure au cours du temps une absorbance en fonction de la longueur d'onde dans la zone de mesure comprenant le fluide comportant le composé chimique d'intérêt, ainsi que la température de ce fluide dans cette même zone de mesure.

Selon une mise en oeuvre de la variante principale de l'invention, on peut réaliser une mesure de l'absorbance en fonction de la longueur d'onde et de la température dans une zone de mesure en aval de l'échantillon de milieu poreux toutes les dix secondes, de préférence toutes les secondes. De tels pas de temps permettent un suivi en continu (ou encore en ligne, ou encore en temps réel) de l'évolution de la concentration en un composé chimique d'un fluide circulant dans un milieu poreux.

Selon une mise en oeuvre selon laquelle le fluide présent dans le milieu ne comporte qu'un seul composé chimique ou un composé chimique additionnel dont le spectre d'absorption est dissocié du spectre d'absorption du composé chimique d'intérêt, la mesure de l'absorbance en fonction de la longueur d'onde conduit directement au spectre d'absorption du composé chimique d'intérêt.

Selon une mise en oeuvre selon laquelle le fluide présent dans le milieu comporte un ou des composés chimiques additionnels par rapport au composé chimique d'intérêt, dont les spectres d'absorption se superposent au moins en partie au spectre d'absorption du composé chimique d'intérêt et dont la concentration est connue et invariante dans le temps, on peut réaliser un prétraitement de la mesure de l'absorbance en fonction de la longueur d'onde tel que décrit ci-dessus afin d'obtenir le spectre d'absorption du composé chimique d'intérêt.

La figure 5 illustre une pluralité de spectres d'absorption SN mesurés au cours de l'étape 3.1) pour une température de fluide mesurée variable par palier ($T_m$ = 17, 26, 40, 50, 60 et 70 °C) et pour une succession de pas de temps. Cette figure présente en outre la superposition de la droite DI déterminée au cours de l'étape 1.2) décrite ci-dessus et qui est utilisée dans l'étape 3.2) décrite ci-dessous.

Ainsi, quelle que soit la température, à l'issue de cette étape, on obtient une pluralité de spectres d'absorption relatifs au composé chimique d'intérêt, chaque spectre d'absorption étant associé à une température et correspondant à un pas de temps de la succession de pas de temps.

### 3.2) Détermination de la concentration en le composé chimique pour chaque pas de temps

Au cours de cette étape, pour chacun des spectres d'absorption correspondant chacun à un pas de temps, on détermine une valeur d'absorbance à l'intersection entre la courbe définie à l'étape 1.2) et le spectre d'absorption considéré. Puis, au moyen du modèle de l'évolution de l'absorbance en fonction de la concentration et de la température, à partir de cette valeur d'absorbance et de la valeur de température mesurée au même pas de temps, on déduit la concentration en le composé chimique d'intérêt pour le pas de temps considéré.

Autrement dit, au cours de cette étape, on recherche l'intersection entre la courbe définie à l'étape 1.2) et chaque spectre d'absorption mesuré à l'étape 3.1), et à partir de la valeur d'absorbance à cette intersection et de la mesure de la température du fluide, on en déduit la concentration en le composé chimique pour ce pas de temps en utilisant le modèle déterminé à l'étape 2), notamment l'équation (3) décrite ci-dessus.

Cette étape peut être illustrée à l'aide de la figure 5, qui présente la superposition sur l'ensemble des spectres d'absorption mesurés à l'étape 3.1) de la droite DI déterminée au cours de l'étape 1.2). Pour chaque spectre mesuré à un pas de temps donné, on détermine la valeur de l'absorbance à l'intersection de la droite DI et du spectre considéré, et au moyen du modèle selon l'équation (3) décrite ci-dessus, qui fait appel aux fonctions Ma(T) et Mb(T) déterminées à l'issue de l'étape 2), et en connaissant la température du fluide, on déduit la valeur de la concentration correspondant à cette valeur d'absorbance. On obtient alors une valeur de concentration pour le pas de temps considéré.

En répétant cette opération pour une succession de pas de temps, on obtient une courbe telle qu'illustrée sur la figure 6, qui présente l'évolution dans le temps t (en minutes) de la concentration C en g/l en le composé chimique d'intérêt, chaque pas de temps étant caractérisé par la température de fluide mesurée Tm en °C.

Il est bien clair que les étapes 3.1) et 3.2) peuvent être mises en œuvre à la fin de chaque pas de temps, pour une détermination de la concentration en le composé chimique d'intérêt en temps réel.

Au moins une partie des étapes du procédé selon l'invention, notamment les étapes 1.2) et/ou 1.3) et/ou 2) et/ou 3.2), peuvent être mises en œuvre au moyen d'un équipement (par exemple un poste de travail informatique, c'est-à-dire un ordinateur) comprenant des moyens de traitement des données (un processeur) et des moyens de stockage de données (une mémoire, en particulier un disque dur), ainsi qu'une interface d'entrée et de sortie pour saisir des données et restituer des résultats.

En outre, l'invention concerne un produit programme d'ordinateur téléchargeable depuis un réseau de communication et/ou enregistré sur un support lisible par ordinateur et/ou exécutable par un processeur, comprenant des instructions de code de programme pour la mise en œuvre au moins des étapes 1.2) et/ou 1.3) et/ou 2) et/ou 3.2) du procédé tel que décrit ci-dessus, lorsque ledit programme est exécuté sur un ordinateur.

L'invention concerne en outre un système pour déterminer une évolution dans le temps d'une concentration en un composé chimique d'un fluide, le système comprenant une source lumineuse, un spectromètre, un capteur de température, ainsi que des moyens pour le traitement et l'analyse de mesures réalisées par le spectromètre, le système étant destiné à la mise en œuvre du procédé tel que décrit ci-dessus.

**Exemples**

Les caractéristiques et avantages du procédé selon l'invention apparaîtront plus clairement à la lecture des exemples d'application ci-après.

**Premier exemple**

Ce premier exemple d'application s'inscrit dans le domaine des géosciences, et vise plus particulièrement à suivre l'évolution dans le temps d'un traceur en sortie d'un milieux poreux. Afin de vérifier la faisabilité du procédé de dosage en conditions hautes températures expliqué ci-dessus, un traceur passif (pas d'interaction chimique avec le milieu) a été choisi. Il s'agit d'une solution d'iodure de potassium (KI) pure dissoute dans de l'eau purifiée. Le milieu poreux considéré est une roche de type carbonate. L'échantillon de roche a un diamètre de 1 cm, une longueur de 2,4 cm, et une perméabilité de 280 mD. Lors de cette expérience de dosage en sortie de la roche, une solution de concentration $c_0$ connue ($c_0$ =5 g/L) en KI est injectée à débit constant 0.1 ml/min dans une roche initialement saturée avec cette même solution.

Le montage expérimental pour cette mise en œuvre du procédé selon l'invention est celui présenté en figure 2b. Ce montage expérimental comprend un système de mesure optique, formé par une source lumineuse SL, pour cet exemple émettant dans les UV (l'iodure de potassium absorbant dans cette gamme de longueur d'ondes) et un spectromètre SP pour mesurer une intensité lumineuse, pour cet exemple dans les UV.

La cellule de mesure CE1 disposée en amont de l'échantillon permet de vérifier la stabilité de la solution injectée dans l'échantillon, alors que la cellule de mesure CE2 disposée en sortie de l'échantillon a pour but de doser les effluents en sortie du milieu. Les cellules de mesure CE1, CE2 sont reliées chacune à la source lumineuse SL et au spectromètre SP par des fibres optiques F de 400 μm de cœur. Le système de circulation de fluide comprend en outre un by-pass BP pour contourner l'échantillon de roche, contrôlé par une vanne V, afin de permettre la mise en œuvre de l'étape 1.1) décrite ci-dessus pour chaque cellule de mesure CE1, CE2 en même temps. Lors de cette expérience, la température du bain thermostaté BT a été d'abord fixée à 17°C puis a varié par palier le long de l'injection jusqu'à atteindre 70 °C. La pression de ligne quant à elle a été maintenue constante à 3 bar.

La mise en œuvre du procédé selon l'invention pour cet exemple d'application, au moyen du montage expérimental décrit ci-dessus, est la suivante :

**a) Construction d'un modèle de l'évolution de l'absorbance en fonction de la concentration et de la température** : pour une température prédéfinie $T_{C1}$ du bain thermostaté, six solutions comprenant de l'iodure de potassium KI à des concentrations connues et couvrant la gamme de concentrations [0 ; $8c_0$] sont injectées une à une dans les cellules de mesure CE1, CE2, sans passer par le milieu poreux disposé dans le porte-échantillon PE, au moyen du by-pass BP. Dans ce cas, la concentration est la même dans les deux cellules de mesure CE1, CE2. Pour chacune des solutions et sur chaque cellule de mesure CE1, CE2, on mesure l'intensité transmise I. L'absorbance associée à la concentration en place est ensuite déduite à partir de I et de $I_0$ qui est l'intensité mesurée préalablement sur un fluide de référence, ici de l'eau purifiée (eau MQ). Par exemple, on peut, à cette fin, saturer le milieu poreux et les cellules de mesure CE1, CE2 avec le fluide de référence qui est ici la solution d'eau MQ. L'intensité $I_0$ associée à cette solution peut être alors mesurée dans les deux cellules de mesure CE1, CE2.

La figure 3, déjà décrite ci-dessus, présente les six spectres d'absorption S1, S6 mesurés pour chaque concentration, ainsi que la droite DI définie lors de l'application de l'étape 1.2) décrite ci-dessus, intersectant les spectres d'absorption S1, S6 en un seul point et de manière qu'une seule valeur d'absorbance corresponde à une seule valeur de longueur d'onde et réciproquement.

La figure 4a présente le modèle intermédiaire MTc1 de l'évolution de l'absorbance pour une première température prédéfinie Tc1=50°C en fonction de la concentration C (en échelle logarithmique) déterminé par régression à partir des valeurs d'absorbance à l'intersection de la droite DI avec les spectres d'absorption S1, S6, ainsi que des concentrations associées à chaque spectre S1, S6.

Les étapes 1.1) à 1.3) du procédé selon l'invention sont répétées pour quatre autres températures prédéfinies Tc2, Tc3, Tc4, et Tc5 en considérant toujours les mêmes six solutions d'iodure de potassium de concentrations connues. La figure 4b présente les modèles intermédiaires de l'évolution de l'absorbance A en fonction de la concentration C (en échelle logarithmique) associés à chacune des températures Tc1 , Tc2, Tc3, Tc4, et Tc5. Comme pour le modèle intermédiaire MTc1, chaque modèle intermédiaire MTci associé à la température prédéfinie Tci (i=2, 3, 4, 5) a été déterminé par régression linéaire à partir des valeurs d'absorbance à l'intersection de la droite DI avec les spectres d'absorption S1, S6, obtenus à la température Tci ainsi que des concentrations associées à chaque spectre S1, S6.

La figure 4c présente l'évolution des coefficients MaTc (pente) et MbTc (ordonnées à l'origine) des cinq modèles intermédiaires MTci (i=1, 2, 3, 4, 5) de la figure 4b en fonction de la température prédéfinie Tc. On détermine alors par régression deux fonctions Ma(T) et Mb(T) respectivement à partir des différentes valeurs des coefficients MaTc et des modèles intermédiaires MTci (i=1, 2, 3, 4, 5) déterminés pour leur température prédéfinie respective Tci (i=1, 2, 3, 4, 5).

Le modèle final d'évolution de l'absorbance en fonction de la concentration et de la température est alors obtenu selon la formule de l'équation (3) décrite ci-dessus, au moyen des deux fonctions Ma(T) et Mb(T) ainsi déterminées.

**b) Suivi de l'évolution de la concentration du traceur en sortie de la roche** : Au moyen du système de circulation de fluide du montage expérimental, une solution KI de concentration connue $c_0$ = 5 g/l est injectée en continu à débit constant (0,1 cc/min pour cet exemple) dans la roche. L'absorbance est alors déterminée en continu dans les deux cellules de mesures CE1, CE2, la mesure dans CE1 servant uniquement à la validation de la concentration en entrée de l'échantillon. La figure 5 déjà décrite ci-dessus présente les spectres d'absorption SN mesurés au niveau de la cellule de mesure CE2 en sortie du milieu poreux, ainsi que la droite DI déjà présentée en figure 3. Elle montre une évolution des spectres au cours du temps due à l'évolution de la température. La figure 6 illustre l'évolution au cours du temps t de la concentration C déterminée en sortie de l'échantillon, à l'issue de l'application de l'étape 3.2 du procédé selon l'invention. On peut observer que le fluide sortant de l'échantillon de roche et traversant la cellule de mesure aval CE2 présente une concentration en KI (dans la gamme de longueurs d'onde d'intérêt) constante et comme attendu égale à $c_0$ qui est la concentration en KI du fluide injecté. A noter que dans cet exemple d'application, on utilise les mesures réalisées au niveau de la cellule de mesure amont CE1 pour vérifier que le fluide traversant la cellule amont CE1 a une concentration en KI

constante (égale à $c_0$).

**Deuxième exemple**

Ce deuxième exemple d'application s'inscrit également dans le domaine des géosciences, et vise plus particulièrement à démontrer la validité du procédé dans des conditions de haute pression.

Pour cet exemple, le milieu poreux, le montage expérimental, le fluide étudié et le débit d'injection sont identiques à l'exemple précédent. La température du bain (et donc du fluide) a été maintenue à 50°C. Seule la pression de ligne (c'est-à-dire la pression du fluide circulant dans l'échantillon) a été modifiée au cours de l'injection.

La figure 7 présente les spectres d'absorption SN mesurés au niveau de la cellule de mesure CE2 en sortie du milieu poreux, ainsi que la droite DI déjà présentée sur la figure 3. Cette figure montre que quelle que soit la pression de ligne, tous les spectres mesurés au cours de l'injection sont superposés.

La figure 8 illustre l'évolution au cours du temps t (en minutes) de la concentration C déterminée en sortie de l'échantillon, à l'issue de l'application de l'étape 3.2 du procédé selon l'invention. Cette figure présente également l'évolution de la température du fluide Tf et de la pression du fluide Pf en fonction du temps t. On peut observer que le fluide sortant de l'échantillon de roche et traversant la cellule de mesure aval CE2 présente une concentration en KI (dans la gamme de longueurs d'onde d'intérêt) constante, et donc non dépendante de la pression de ligne.

Ainsi, le procédé selon l'invention permet, grâce à une méthode simple et rapide, un suivi en temps réel de l'évolution de la concentration en un composé chimique dans un fluide, et ce dans une large gamme de concentrations et pour une température de fluide pouvant varier dans le temps. En effet, le procédé selon l'invention permet une mesure de la concentration en un composé chimique :

- Même lorsque le composé chimique est présent en forte concentration, notamment dans une concentration au-delà de laquelle la loi de Beer-Lambert ne peut plus être appliquée.
- Quelle que soit la température du fluide dans la gamme, à condition qu'elle soit connue.
- Et ce, qui plus est, quelle que soit la pression de ligne, comme démontré dans le deuxième exemple d'application ci-dessus.

De plus, le procédé selon l'invention permet, une fois le modèle de l'évolution de l'absorbance en fonction de la concentration et de la température construit, de déterminer la concentration en le composé chimique d'intérêt après chaque acquisition d'un spectre d'absorption, et ainsi de suivre en ligne l'évolution de la concentration en le composé chimique d'intérêt.

## Revendications

1. Procédé pour déterminer une évolution dans le temps d'une concentration (C) en un composé chimique d'un fluide (FL) en circulation dans une zone de mesure (ZM), au moyen au moins d'un système de mesure optique (SMO, SL, SP) pour mesurer une absorbance (A) en fonction d'une longueur d'onde (L) dudit fluide (FL) et d'un capteur de température (ST) pour mesurer une température dudit fluide (FL), ledit composé chimique dudit fluide (FL) étant l'unique composé chimique dudit fluide (FL) ou l'unique composé chimique dudit fluide (FL) dont ladite concentration (C) varie ou l'unique composé chimique dudit fluide (FL) ayant une absorbance (A) non nulle dans une gamme de longueur d'ondes absorbées par ledit composé chimique, caractérisé en que :

   A) pour chaque température prédéfinie d'une pluralité de températures prédéfinies, on construit un modèle intermédiaire (MTc1, MTc5) de l'évolution de ladite absorbance (A) en fonction de ladite concentration (C) pour ladite température prédéfinie de la manière suivante :

      i) au moyen au moins dudit système de mesure optique (SMO, SL, SP), on mesure une absorbance (A) en fonction de la longueur d'onde (L) pour une pluralité d'échantillons dudit fluide (FL) à ladite température prédéfinie et ayant des concentrations en ledit composé chimique distinctes, et on obtient une première pluralité de spectres d'absorption (S1, S6) relatifs audit composé chimique correspondant chacun à une desdites concentrations en ledit composé chimique ;
      ii) on définit une courbe (DI) intersectant chacun desdits spectres d'absorption (S1, S6) de ladite première pluralité de spectres d'absorption en un seul point d'intersection et telle que ladite courbe (DI) soit une fonction bijective de ladite absorbance (A) et de ladite longueur d'onde (L) ;
      iii) on construit ledit modèle intermédiaire (MTc1, MTc5) de l'évolution de ladite absorbance (A) en fonction de ladite concentration (C) pour ladite température prédéfinie au moyen d'une régression linéaire appliquée à des premières valeurs d'absorbance auxdits points d'intersection entre ladite courbe (DI) et chacun desdits spectres d'absorption (S1, S6) de ladite première pluralité de spectres d'absorption, et à ladite concentration (C) correspondant à chacun desdits spectres d'absorption (S1, S6) de ladite première pluralité de spectres d'absorption ;

   B) à partir de coefficients directeurs (MaTc) et d'ordonnées à l'origine (MbTc) déterminés pour chacun desdits modèles intermédiaires (MTc1, MTc5) de l'évolution de ladite absorbance (A) en fonction de ladite concentration (C) construits pour chacune desdites températures prédéfinies, on détermine par régression linéaire une première fonction Ma(T) représentative d'une variation dudit coefficient directeur (MaTC) en fonction de ladite température (T) et une deuxième fonction Mb(T) représentative d'une variation de ladite ordonnée à l'origine (MbTC) en fonction de ladite température (T), et on construit ledit modèle (M) de l'évolution de ladite absorbance (A) en fonction de ladite concentration (C) et de ladite température (T) selon une formule du type :

$$A = Ma(T).\ln(C) + Mb(T)$$

où T est ladite température dudit fluide (FL) et C est ladite concentration dudit fluide (FL) ;
   C) on détermine une évolution dans le temps d'une concentration (C) en ledit composé chimique dudit fluide (FL) circulant dans ladite zone de mesure (ZM) de la manière suivante :

   a) au moyen au moins dudit système de mesure optique (SMO, SL, SP) et dudit capteur de température (ST), on mesure dans ladite zone de mesure (ZM) respectivement une absorbance (A) en fonction de la longueur d'onde (L) et une température (TM) pour une succession de pas de temps et on obtient une deuxième pluralité de spectres d'absorption (SN) relatifs audit composé chimique correspondant chacun à un pas de temps ainsi qu'une température mesurée (TM) pour chaque pas de temps ; et
   b) pour chacun desdits spectres d'absorption (SN) de ladite deuxième pluralité de spectres d'absorption (SN), on détermine une deuxième valeur d'absorbance à l'intersection entre ladite courbe (DI) et ledit spectre d'absorption (SN), et, au moyen dudit modèle de l'évolution de ladite absorbance (A) en fonction de ladite concentration (C) et de ladite température mesurée (T), et à partir de ladite deuxième valeur d'absorbance et de ladite température mesurée (TM), on déduit ladite concentration (C) en ledit composé chimique pour ledit pas de temps.

**2.** Procédé selon l'une des revendications précédentes, dans lequel ladite courbe (DI) est une droite.

**3.** Procédé selon l'une des revendications précédentes, dans lequel ledit système de mesure optique (SMO, SL, SP) comprend au moins une source lumineuse (SL) pour émettre un rayonnement dans au moins dans une gamme de longueur d'ondes prédéterminée, et un spectromètre (SP) pour mesurer une intensité lumineuse dudit rayonnement transmis au travers de ladite zone de mesure (ZM) au moins dans ladite gamme de longueur d'ondes prédéfinie.

**4.** Procédé selon la revendication 3, dans lequel ledit système de mesure optique (SMO, SL, SP) comprend en outre au moins une cellule de mesure (CE, CE1, CE2, CE3) reliée à ladite source lumineuse (SL) et audit spectromètre (SP), et dans laquelle se trouve ledit fluide.

**5.** Procédé selon l'une des revendications précédentes, dans lequel, lorsque ledit composé chimique est l'unique composé chimique dudit fluide (FL) dont ladite concentration (C) varie et en l'absence d'une étape de calibration dudit système de mesure optique (SMO, SL, SP) au moyen d'un fluide de référence correspondant audit fluide à l'exclusion dudit composé chimique, on applique une étape de prétraitement aux mesures d'absorbance (A) en fonction de la longueur d'onde (L) pour déterminer un spectre d'absorption dudit composé chimique, comprenant au moins une soustraction dudit spectre d'absorption dudit composé chimique additionnel préalablement enregistré.

**6.** Procédé selon l'une des revendications précédentes, dans lequel ladite zone de mesure (ZM) est disposée en aval d'un milieu poreux, tel qu'un échantillon d'une roche d'une formation souterraine, dans lequel circule ledit fluide (FL).

**7.** Procédé selon la revendication 6, dans lequel ledit composé chimique est choisi parmi la liste suivante : un tensio-actif, un sel, un composé hydrocarboné, un polymère.

**8.** Procédé selon l'une des revendications 6 à 7, dans lequel ledit procédé est mis en œuvre au moyen en outre d'un système de circulation de fluide (P, BP, V, PE, BT) pour faire circuler ledit fluide au moins dans ledit milieu poreux et ladite zone de mesure, ledit système de circulation de fluide (P, BP, V, PE) comprenant une pompe (P), de préférence une pompe (P) apte à délivrer un débit avec une haute précision, une cellule porte échantillon (PE) dans laquelle est disposé ledit milieu poreux, et de préférence un bain thermostaté (BT) ou une étuve pour contrôler la température dudit fluide dans ladite zone de mesure.

**9.** Système pour déterminer une évolution dans le temps d'une concentration en un composé chimique d'un fluide, ledit système comprenant une source lumineuse (SL), un spectromètre (SP), et des moyens pour le traitement et l'analyse de mesures réalisées par ledit spectromètre (SP), ledit système étant apte à la mise en œuvre du procédé selon l'une des revendications précédentes.

**10.** Produit programme d'ordinateur téléchargeable depuis un réseau de communication et/ou enregistré sur un support lisible par ordinateur et/ou exécutable par un processeur, comprenant des instructions de code de programme pour la mise en œuvre des étapes ii, et/ou iii), et/ou B), et/ou b), du procédé selon l'une des revendications 1 à 8, lorsque ledit programme est exécuté sur un ordinateur.

**Patentansprüche**

**1.** Verfahren zur Bestimmung eines zeitlichen Verlaufs einer Konzentration (C) einer chemischen Verbindung eines in einem Messbereich (ZM) zirkulierenden Fluids (FL) mittels mindestens eines optischen Messsystems (SMO, SL, SP) zur Messung einer Extinktion (A) als Funktion einer Wellenlänge (L) des Fluids (FL) und eines Temperatursensors (ST) zur Messung einer Temperatur des Fluids (FL), wobei es sich bei der chemischen Verbindung des Fluids (FL) um die einzige chemische Verbindung des Fluids (FL) oder die einzige chemische Verbindung des Fluids (FL), deren Konzentration (C) sich verändert, oder die einzige chemische Verbindung des Fluids (FL) mit einer Extinktion (A) ungleich Null in einem von der chemischen Verbindung absorbierten Wellenlängenbereich handelt, **dadurch gekennzeichnet, dass**:

A) für jede vordefinierte Temperatur einer Mehrzahl von vordefinierten Temperaturen ein Zwischenmodell (MTc1, MTc5) des Verlaufs der Extinktion (A) als Funktion der Konzentration (C) für die vordefinierte Temperatur auf die folgende Weise konstruiert wird:

i) mittels zumindest des optischen Messsystems (SMO, SL, SP) wird eine Extinktion (A) als Funktion der Wellenlänge (L) für eine Mehrzahl von Proben des Fluids (FL) bei der vordefinierten Temperatur und mit verschiedenen Konzentrationen der chemischen Zusammensetzung gemessen, und es wird eine erste Mehrzahl von Absorptionsspektren (S1, S6) in Bezug auf die chemische Zusammensetzung erhalten, die jeweils einer der Konzentrationen der chemischen Verbindung entsprechen;

ii) es wird eine Kurve (DI), die jedes der Absorptionsspektren (S1, S6) der ersten Mehrzahl von Absorptionsspektren an einem einzigen Schnittpunkt schneidet, definiert, und so, dass die Kurve (DI) eine bijektive Funktion der Extinktion (A) und der Wellenlänge (L) ist;

iii) das Zwischenmodell (MTc1, MTc5) des Verlaufs der Extinktion (A) als Funktion der Konzentration (C) für die vordefinierte Temperatur wird mittels einer linearen Regression konstruiert, die auf die ersten Extinktionswerte bei den Schnittpunkten zwischen der Kurve (DI) und jedem der Absorptionsspektren (S1, S6) der ersten Mehrzahl von Absorptionsspektren und bei der Konzentration (C) angewandt wird, die jedem der Absorptionsspektren (S1, S6) der ersten Mehrzahl von Absorptionsspektren entspricht;

B) ausgehend von den Steigungen (MaTc) und den y-Achsenabschnitten (MbTc), die für jedes der Zwischenmodelle (MTc1, MTc5) des Verlaufs der Extinktion (A) als Funktion der Konzentration (C) bestimmt werden, die für jede der vordefinierten Temperaturen konstruiert wurden, durch lineare Regression eine erste Funktion Ma(T), die für eine Änderung der Steigung (MaTC) als Funktion der Temperatur (T) steht, und eine zweite Funktion Mb(T), die für eine Änderung des y-Achsenabschnitts (MbTC) als Funktion der Temperatur (T) steht, bestimmt werden, und das Modell (M) des Verlaufs der Extinktion (A) als Funktion der Konzentration (C) und der Temperatur (T) gemäß einer Formel des Typs:

$$A = Ma(T).ln(C) + Mb(T)$$

bestimmt wird, wobei T die Temperatur des Fluids (FL) ist und C die Konzentration des Fluids (FL) ist;

C) ein zeitlicher Verlauf einer Konzentration (C) der chemischen Verbindung des in dem Messbereich (ZM) zirkulierenden Fluids (FL) auf die folgende Weise bestimmt wird:

a) im Messbereich (ZM) wird mittels des mindestens einen optischen Messsystems (SMO, SL, SP) und des Temperatursensors (ST) jeweils eine Extinktion (A) als Funktion der Wellenlänge (L) und eine Temperatur (TM) für eine Abfolge von Zeitschritten gemessen, und es wird eine zweite Mehrzahl von Absorptionsspektren (SN) in Bezug auf die chemische Zusammensetzung erhalten, die jeweils einem Zeitschritt sowie einer für jeden Zeitschritt gemessenen Temperatur (TM) entsprechen; und

b) für jedes der Absorptionsspektren (SN) der zweiten Mehrzahl von Absorptionsspektren (SN) wird ein zweiter Extinktionswert am Schnittpunkt zwischen der Kurve (DI) und dem Absorptionsspektrum (SN) bestimmt, und mittels des Modells des Verlaufs der Extinktion (A) als Funktion der Konzentration (C) und der gemessenen Temperatur (T) und ausgehend von dem zweiten Extinktionswert und der gemessenen Temperatur (TM) wird die Konzentration (C) der chemischen Zusammensetzung für den Zeitschritt abgeleitet.

2. Verfahren nach einem der vorhergehenden Ansprüche, wobei die Kurve (DI) eine Gerade ist.

3. Verfahren nach einem der vorhergehenden Ansprüche, wobei das optische Messsystem (SMO, SL, SP) mindestens eine Lichtquelle (SL) zum Ausstrahlen einer Strahlung in zumindest einem vorbestimmten Wellenlängenbereich und ein Spektrometer (SP) zum Messen einer Lichtstärke der durch den Messbereich (ZM) durchgelassenen Strahlung zumindest im vordefinierten Wellenlängenbereich umfasst.

4. Verfahren nach Anspruch 3, wobei das optische Messsystem (SMO, SL, SP) zusätzlich mindestens eine Messzelle (CE, CE1, CE2, CE3) umfasst, die mit der Lichtquelle (SL) und dem Spektrometer (SP) verbunden ist und in der sich das Fluid befindet.

5. Verfahren nach einem der vorhergehenden Ansprüche, wobei, wenn es sich bei der chemischen Verbindung um die einzige chemische Verbindung des Fluids (FL) handelt, deren Konzentration (C) sich verändert, und ohne einen Schritt einer Kalibrierung des optischen Messsystems (SMO, SL, SP) mittels eines Referenzfluids, das mit Ausnahme der chemischen Verbindung dem Fluid entspricht, ein Vorbehandlungsschritt auf die Extinktionsmessungen (A) als Funktion der Wellenlängen (L) angewandt wird, um ein Absorptionsspektrum der chemischen Verbindung zu bestimmen, der mindestens eine Subtraktion des zuvor aufgezeichneten Absorptionsspektrums der zusätzlichen chemischen Verbindung umfasst.

6. Verfahren nach einem der vorhergehenden Ansprüche, wobei der Messbereich (ZM) hinter einem porösen Medium, wie einer Probe eines Gesteins einer unterirdischen Formation, angeordnet ist, in der das Fluid (FL) zirkuliert.

7. Verfahren nach Anspruch 6, wobei die chemische Verbindung aus der folgenden Liste ausgewählt ist: ein Tensid, ein Salz, eine Kohlenwasserstoffverbindung, ein Polymer.

8. Verfahren nach einem der Ansprüche 6 bis 7, wobei das Verfahren außerdem mittels eines Fluidzirkulationssystems (P, BP, V, PE, BT) durchgeführt wird, um das Fluid zumindest in dem porösen Medium und dem Messbereich zirkulieren zu lassen, wobei das Fluidzirkulationssystem (P, BP, V, PE, BT) eine Pumpe (P), vorzugsweise eine Pumpe (P), die zur Förderung eines Förderstroms mit hoher Präzision fähig ist, eine Probenhalterzelle (PE), in der das poröse Medium angeordnet ist, und vorzugsweise ein temperiertes Bad (BT) oder einen Ofen zur Steuerung der Temperatur des Fluids in dem Messbereich umfasst.

9. System zur Bestimmung eines zeitlichen Verlaufs einer Konzentration einer chemischen Verbindung eines Fluids, wobei das System eine Lichtquelle (SL), ein Spektrometer (SP) und Mittel zur Behandlung und Analyse von Messungen umfasst, die mit dem Spektrometer (SP) erfolgen, wobei das System zur Durchführung des Verfahrens nach einem der vorhergehenden Ansprüche geeignet ist.

10. Computerprogrammprodukt, das aus einem Kommunikationsnetzwerk heruntergeladen werden kann und/oder auf einem computerlesbaren Medium aufgezeichnet ist und/oder durch einen Prozessor ausführbar ist und das Programmcodeanweisungen für die Umsetzung der Schritte ii) und/oder iii) und/oder B und/oder b) des Verfahrens nach einem der Ansprüche 1 bis 8 umfasst, wenn das Programm auf einem Computer ausgeführt wird.

**Claims**

1. Method for determining a change over time in a concentration (C) of a chemical compound in a fluid (FL) flowing through a measuring zone (ZM), by means of at least one optical measuring system (SMO, SL, SP) for measuring an absorbance (A) as a function of a wavelength (L) of said fluid (FL) and of a temperature sensor (ST) for measuring a temperature of said fluid (FL), said chemical compound in said fluid (FL) being the only chemical compound in said fluid (FL) or the only chemical compound in said fluid (FL) the concentration (C) of which varies or the only chemical compound in said fluid (FL) having a non-zero absorbance (A) in a range of wavelengths absorbed by said chemical compound, **characterized in that**:

A) for each predefined temperature of a plurality of predefined temperatures, an intermediate model (MTc1, MTc5) of the change in said absorbance (A) as a function of said concentration (C) is constructed for said predefined temperature as follows:

i) at least by means of said optical measuring system (SMO, SL, SP), an absorbance (A) as a function of wavelength (L) is measured for a plurality of samples of said fluid (FL) at said predefined temperature and having different concentrations of said chemical compound, and a first plurality of absorption spectra (S1, S6) relating to said chemical compound and each corresponding to one of said concentrations of said chemical compound are obtained;
ii) a curve (DI) is defined that intersects each of said absorption spectra (S1, S6) of said first plurality of absorption spectra at a single point of intersection and that is such that said curve (DI) is a bijective function of said absorbance (A) and of said wavelength (L);
iii) said intermediate model (MTc1, MTc5) of the change in said absorbance (A) as a function of said concentration (C) for said predefined temperature is constructed by means of a linear regression applied to first absorbance values at said points of intersection between said curve (DI) and each of said absorption spectra (S1, S6) of said first plurality of absorption spectra, and to said concentration (C) corresponding to each of said absorption spectra (S1, S6) of said first plurality of absorption spectra;

B) from slopes (MaTc) and intercepts (MbTc) determined for each of said intermediate models (MTc1, MTc5) of the change in said absorbance (A) as a function of said concentration (C) constructed for each of said predefined temperatures, a first function Ma(T) representative of a variation in said slope (MaTc) as a function of said temperature (T) and a second function Mb(T) representative of a variation in said intercept (MbTc) as a function of said temperature (T) are determined by linear regression, and said model (M) of the change in said absorbance (A) as a function of said concentration (C) and of said temperature (T) is constructed according to a formula of the type:

```
A = Ma(T).ln(C) + Mb(T)
```

where T is said temperature of said fluid (FL) and C is said concentration of said fluid (FL);

C) a change over time in a concentration (C) of said chemical compound in said fluid (FL) flowing through said measuring zone (ZM) is determined as follows:

a) by means of at least said optical measuring system (SMO, SL, SP) and of said temperature sensor (ST), an absorbance (A) as a function of wavelength (L) and a temperature (TM), respectively, are measured in said measuring zone (ZM) for a succession of time increments, and a second plurality of absorption spectra (SN) relating to said chemical compound are obtained each corresponding to one time increment and to one measured temperature (TM) for each time increment; and

b) for each of said absorption spectra (SN) of said second plurality of absorption spectra (SN), a second absorbance value is determined at the intersection between said curve (DI) and said absorption spectrum (SN), and, by means of said model of the change in said absorbance (A) as a function of said concentration (C) and of said measured temperature (T), and based on said second absorbance value and on said measured temperature (TM), said concentration (C) of said chemical compound is deduced for said time increment.

2. Method according to one of the preceding claims, wherein said curve (DI) is a straight line.

3. Method according to either of the preceding claims, wherein said optical measuring system (SMO, SL, SP) comprises at least one light source (SL) for emitting radiation in at least one predetermined wavelength range, and a spectrometer (SP) for measuring a light intensity of said radiation transmitted through said measuring zone (ZM) at least in said predetermined wavelength range.

4. Method according to Claim 3, wherein said optical measuring system (SMO, SL, SP) further comprises at least one measurement cell (CE, CE1, CE2, CE3) connected to said light source (SL) and to said spectrometer (SP), and in which said fluid is found.

5. Method according to any of the preceding claims, wherein, when said chemical compound is the only chemical compound in said fluid (FL) the concentration (C) of which varies and in the absence of a step of calibrating said optical measuring system (SMO, SL, SP) by means of a reference fluid corresponding to said fluid excluding said chemical compound, a pre-processing step is applied to the absorbance measurements (A) as a function of wavelength (L) to determine an absorption spectrum of said chemical compound, comprising at least one subtraction of said absorption spectrum of said additional chemical compound recorded beforehand.

6. Method according to any of the preceding claims, wherein said measuring zone (ZM) is placed downstream of a porous medium, such as a rock sample from an underground formation, through which said fluid (FL) flows.

7. Method according to Claim 6, wherein said chemical compound is selected from the following list: a surfactant, a salt, a hydrocarbon compound, a polymer.

8. Method according to either of Claims 6 and 7, wherein said method is implemented by means furthermore of a system (P, BP, V, PE, BT) for making fluid flow, which is used to make said fluid flow at least through said porous medium and said measuring zone, said system (P, BP, V, PE) for making fluid flow comprising a pump (P), preferably a pump (P) capable of delivering a flow rate with a high precision, a sample-holding cell (PE) in which said porous medium is placed, and preferably a thermostatically controlled bath (BT) or an oven for controlling the temperature of said fluid in said measuring zone.

9. System for determining a change over time in a concentration of a chemical compound in a fluid, said system comprising a light source (SL), a spectrometer (SP), and means for processing and analysing measurements made by said spectrometer (SP), said system being capable of implementing the method according to any of the preceding claims.

10. Computer program product downloadable from a communication network and/or recorded on a medium that is readable by computer and/or executable by a processor, comprising program code instructions for implementing step(s) ii, and/or iii), and/or B), and/or b), of the method according to any of Claims 1 to 8, when said program is executed on a computer.

EP 4 495 581 B1

[Fig 1]

[Fig 2a]

20

[Fig 2b]

[Fig 2c]

[Fig 3]

[Fig 4a]

[Fig 4b]

[Fig 4c]

[Fig 5]

[Fig 6]

[Fig 7]

[Fig 8]

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2201215

**Littérature non-brevet citée dans la description**

- **MALIK, M.** ; **CHAN, K. H.** ; **AZIMI, G.** Quantification of nickel, cobalt, and manganese concentration using ultraviolet-visible spectroscopy.. *RSC Advances*, 2021, vol. 11 (45), 28014-28028